(19) Europäisches Patentamt
European Patent Office
Office européen des brevets

(11) EP 1 872 853 A1

(12) EUROPEAN PATENT APPLICATION
published in accordance with Art. 153(4) EPC

(43) Date of publication:
02.01.2008 Bulletin 2008/01

(21) Application number: 06713220.9

(22) Date of filing: 07.02.2006

(51) Int Cl.:
*B01J 31/22* (2006.01) *C07C 2/22* (2006.01)
*C07C 11/113* (2006.01) *C07B 61/00* (2006.01)

(86) International application number:
PCT/JP2006/302077

(87) International publication number:
WO 2006/085531 (17.08.2006 Gazette 2006/33)

(84) Designated Contracting States:
DE FR GB IT

(30) Priority: 10.02.2005 JP 2005034059

(71) Applicant: Mitsui Chemicals, Inc.
Tokyo 105-7117 (JP)

(72) Inventors:
- OKUDA, Jun
52074 (DE)
- SUZUKI, Yasuhiko
aura-shi, Chiba, 2990265 (JP)

(74) Representative: Cresswell, Thomas Anthony
J.A. KEMP & CO.
14 South Square
Gray's Inn
London WC1R 5JJ (GB)

# (54) CATALYST FOR PROPYLENE DIMERIZATION AND DIMERIZATION METHOD

(57) [PROBLEMS] To provide a catalyst for dimerizing propylene which has excellent propylene dimerization activity and a method of producing 4-methyl-1-pentene using the catalyst.

[MEANS FOR SOLVING PROBLEMS] A catalyst for dimerizing propylene which contains a transition metal complex [A], and as needed, at least one compound [B] selected from the group consisting of an organometallic compound (b-1), an organoaluminumoxy compound (b-2), and a compound (b-3) which reacts with the transition metal complex [A] to form an ion pair, and a method of dimerizing propylene using the catalyst.

EP 1 872 853 A1

**Description**

TECHNICAL FIELD

The present invention relates to a catalyst for dimerizing propylene, which contains a transition metal complex, and a method of producing 4-methyl-1-pentene using the catalyst for dimerizing propylene.

BACKGROUND ART

In the past, the dimerization of propylene has been carried out at high temperature and under a high pressure by using a solid catalyst in which an alkali metal such as sodium or potassium is supported on potassium carbonate or graphite. Meanwhile, as the catalyst for dimerizing propylene, which includes a transition metal, there is a well known catalyst of so-called nickel catalyst. The nickel catalyst is expected to reduce the production facilities or production cost because propylene is capable of being dimerized under a very mild reaction condition, but the selectivity for production of 4-methyl-1-pentene is equally low (see Chemical Reviews, 1991, Vol. 91, p. 613). An uranium catalyst (see US Patent No. 4,695,669), and a zirconium catalyst and a hafnium catalyst (see Chemistry Letter, 1991, pp. 1525 - 1528, Organometallics, 1992, Vol. 11, pp. 362 - 369, and Journal of Molecular Catalysis, 1990, Vol. 62, pp. 277 - 287) are known as other catalyst for dimerizing propylene employing a transition metal that gives a high selectivity for 4-methyl-1-pentene. However, the uranium catalyst is problematic in views of safty in respect to an environment in use and treatment after the use, and, in the zirconium and the hafnium catalyst, a balance between catalyst activity and selectivity is not achieved at the same time. Accordingly, a catalyst for dimerizing propylene with high catalytic activity and selectivity has been demanded.

[Patent Document 1] US Patent No. 4,695,669
[Non-Patent Document 1] Chemical Reviews, 1991, Vol. 91, p. 613
[Non-Patent Document 2] Chemistry Letter, 1991, pp. 1525 - 1528
[Non-Patent Document 3] Organometallics, 1992, Vol. 11, pp. 362 - 369
[Non-Patent Document 4] Journal of Molecular Catalysis, 1990, Vol. 62, pp. 277 - 287

DISCLOSURE OF THE INVENTION

Problems to be Solved by the Invention

It is an object of the present invention to provide a catalyst for dimerizing propylene, which contains a transition metal complex and has excellent activity and selectivity for production of 4-methyl-1-pentene, and a method of producing 4-methyl-1-pentene using the catalyst.

Means for Solving the Problems

The present inventors have studied extensively to solve the above-mentioned problems, and as a result, they have found that a catalyst for dimerizing propylene, which contains a transition metal complex having a predetermined structure, has excellent activity and high selectivity for production of 4-methyl-1-pentene and is useful to produce 4-methyl-1-pentene, which has led to completion of the invention.
That is, the invention provides:

[1] a catalyst for dimerizing propylene containing a transition metal complex [A1] which is represented by the following general formula (I):

[Chem.1]

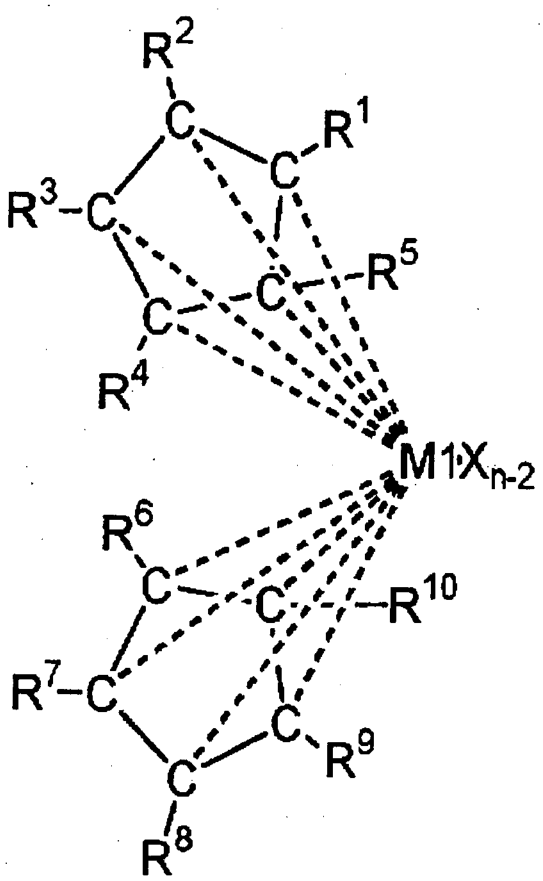

(I)

(wherein $R^1$ to $R^{10}$ may or may not be the same and represent each a halogen atom, a hydrocarbon group, a heterocyclic compound residue, an oxygen-containing group, a nitrogen-containing group, a boron-containing group, an aluminum-containing group, a sulfur-containing group, a phosphorus-containing group, a silicon-containing group, a germanium-containing group or a tin-containing group, while two or more of the $R^1$ to $R^{10}$ may be linked to each other and at least one of the $R^1$ to $R^{10}$ is a group other than a methyl group; M1 represents a transition metal atom of Groups III to XI of the periodic table other than lanthanoid atoms and actinoid atoms; n represents a valence of M1; X represents a hydrogen atom, a halogen atom, a hydrocarbon group, an oxygen-containing group, a sulfur-containing group, a nitrogen-containing group, a boron-containing group, an aluminum-containing group, a phosphorus-containing group, a halogen-containing group, a heterocyclic compound residue, a silicon-containing group, a germanium-containing group or a tin-containing group, while groups represented by X may or may not be the same with each other and they may be linked to each other to form a ring; and in the formula each of dotted lines represents a coordinate bond);

[2] the catalyst for dimerizing propylene described in [1] above, in which the hydrocarbon groups of the $R^1$ to $R^{10}$ of the transition metal complex [A1] represented by the general formula (I) are hydrocarbon groups having 1 to 30 carbon atom(s);

[3] the catalyst for dimerizing propylene described in [1] or [2] above, in which M1 of the transition metal complex [A1] represented by the general formula (I) is a transition metal atom of Group IV or V of the periodic table;

[4] the catalyst for dimerizing propylene described in [1] above, in which $R^1$ to $R^{10}$ of the transition metal complex [A1] that is represented by general formula (I) are hydrocarbon groups having 1 to 20 carbon atom(s) and M1 is a hafnium atom;

[5] the catalyst for dimerizing propylene described in any one of [1] to [4] above, which contains the transition metal complex [A1] represented by the general formula (I), and at least one compound selected from the group [B1] consisting of an organometallic compound (b1-1), an organoaluminumoxy compound (b1-2), and a compound which reacts with the transition metal complex [A1] to form an ion pair (b1-3);

[6] a catalyst for dimerizing propylene containing a transition metal complex [A2] represented by a general formula (II):

[Chem.2]

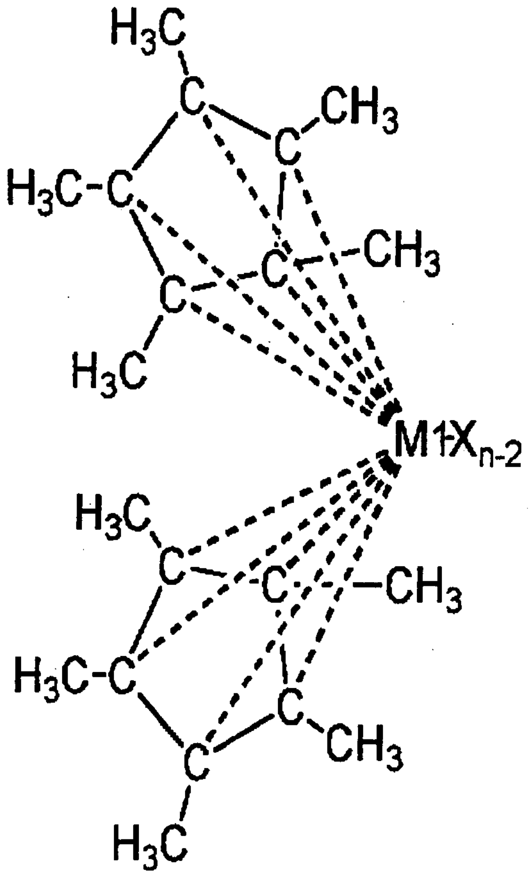

(II)

(wherein, M1 represents a transition metal atom of Groups III to XI of the periodic table other than lanthanoid atoms and actinoid atoms; n represents a valence of M1; X represents a hydrogen atom, a halogen atom, a hydrocarbon group, an oxygen-containing group, a sulfur-containing group, a nitrogen-containing group, a boron-containing group, an aluminum-containing group, a phosphorus-containing group, a halogen-containing group, a heterocyclic compound residue, a silicon-containing group, a germanium-containing group, or a tin-containing group, while groups represented by X may or may not be the same and may be linked to each other to form a ring; and each of dotted lines represents a coordinate bond); and at least one compound [B2] selected from the group consisting of an organometallic compound (b2-1) and a compound (b2-3) which reacts with the transition metal complex [A2] to form an ion pair;

[7] the catalyst for dimerizing propylene according to [6], in which M1 of the transition metal complex [A2] represented by the general formula (II) is a hafnium or zirconium atom;

[8] the catalyst for dimerizing propylene according to any one of [1] to [7] above, further comprising a carrier [C]; and

[9] a method of producing 4-methyl-1-pentene, in which propylene is dimerized in the presence of the catalyst for dimerizing propylene described in any one of [1] to [8] above.

Hereinafter, [A1] and [A2] may be referred as the general term [A] and [B1] and [B2] may be referred as the general term [B]. Furthermore, [b-1] is the general term for [b1-1] and [b2-1].

Effect of the Invention

The invention provides a catalyst for dimerizing propylene, which has high activity. In a method of dimerizing propylene according to the invention, 4-methyl-1-pentene can be produced with high activity and selectivity. Furthermore, when polymerizing 4-methyl-1-pentene in the presence of a generally known polymerization catalyst such as a titanium

catalyst or a metallocene catalyst, the amount of components produced, which inhibit the polymerization, is small in the obtained propylene dimers, and the invention is industrially a lot valuable.

BEST MODE FOR CARRYING OUT THE INVENTION

Hereinafter, a catalyst for dimerizing propylene of the invention and a method of producing 4-methyl-1-pentene using the catalyst thereof will be described in detail.
A catalyst for dimerizing propylene of the invention comprises [A1] a transition metal complex represented by the general formula (I), and, if necessary, [B1] at least one compound selected from the group consisting of (b1-1) an organometallic compound, (b1-2) an organoaluminumoxy compound, and (b1-3) a compound which reacts with the transition metal complex [A1] to form an ion pair.
Generally, it is preferable to perform a dimerization reaction by using a catalyst that contains both the component [A1] and the component [B1].
Furthermore, a catalyst for dimerizing propylene of the invention comprises [A2] a transition metal complex represented by the general formula (II), and at least one compound selected from the group [B2] consisting of (b2-1) an organometallic compound and (b2-3) a compound which reacts with the transition metal complex [A2] to form an ion pair.
Hereinbelow, there may be a case where the compound which reacts with the transition metal complex [A] to form an ion pair is referred to as "ionized ionic compound".
First, a component constituting the catalyst used in the invention will be described.

[Transition metal complex [A]]

The transition metal complexes [A1] and [A2] of the invention are transition metal complexes represented by the following general formulae (I) and (II), respectively.

[Chem.3]

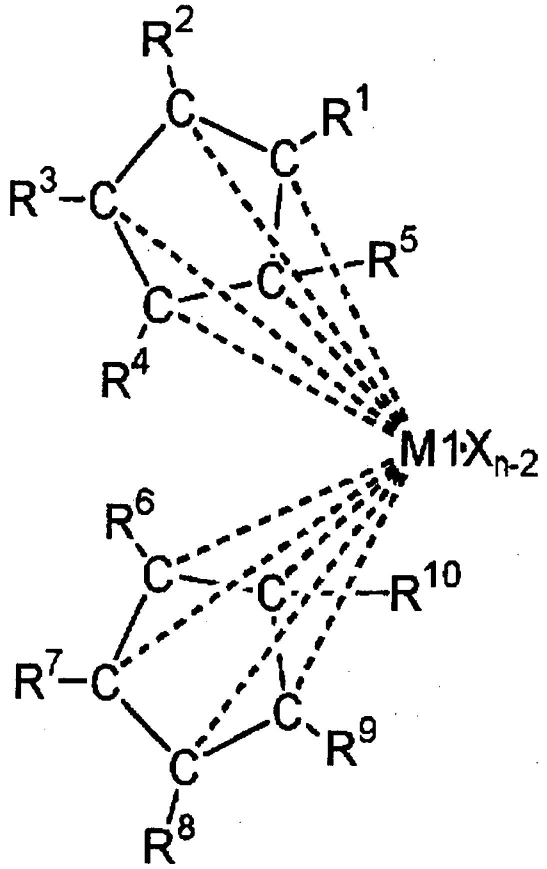

(I)

[Chem.4]

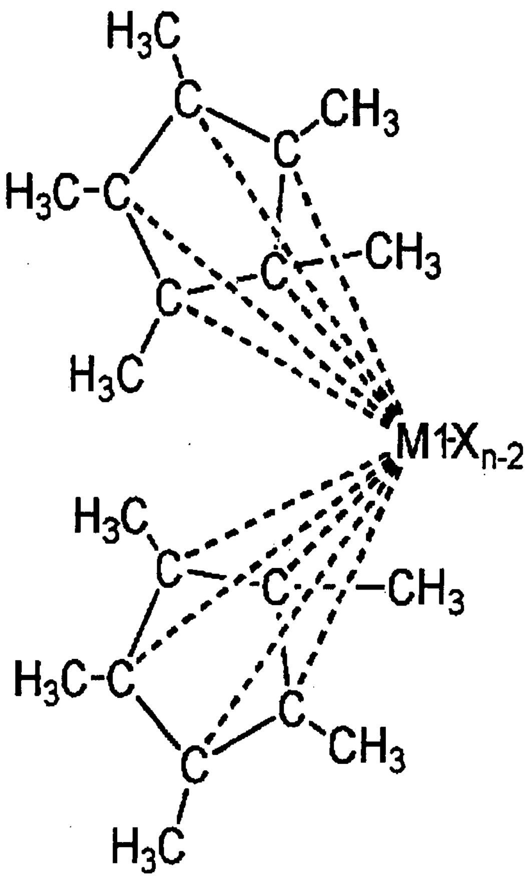

(II)

In general formula (I), the $R^1$ to $R^{10}$ may or may not be the same and represent each a halogen atom, a hydrocarbon group, a heterocyclic compound residue, an oxygen-containing group, a nitrogen-containing group, a boron-containing group, an aluminum-containing group, a sulfur-containing group, a phosphorus-containing group, a silicon-containing group, a germanium-containing group or a tin-containing group, of which two or more may be linked to each other to form a ring.

More specifically, it is preferable that the $R^1$ to $R^{10}$ are each a halogen atom, a hydrocarbon group, a heterocyclic compound residue, a hydrocarbon-substituted silyl group, a hydrocarbon-substituted siloxy group, an alkoxy group, an alkylthio group, an aryloxy group, an arylthio group, an acyl group, an ester group, a thioester group, an amide group, an imide group, an amino group, an imino group, a sulfone ester group, a sulfone amide group, a cyano group, a nitro group, a carboxyl group, a sulfo group, a mercapto group, an aluminum-containing group or a hydroxy group.

Examples of the halogen atom include fluorine, chlorine, bromine and iodine.

Specific examples of the hydrocarbon group include a straight-chained or branched alkyl group having 1 to 30, preferably 1 to 20, more preferably 1 to 10, and most preferably 2 to 10 carbon atom(s) such as methyl, ethyl, n-propyl, isopropyl, n-butyl, isobutyl, sec-butyl, tert-butyl, neopentyl and n-hexyl; a straight-chained or branched alkenyl group having 2 to 30, preferably 2 to 20 carbon atoms such as vinyl, allyl and iso-propenyl; a straight-chained or branched alkynyl group having 2 to 30, preferably 2 to 20 carbon atoms such as ethynyl and propargyl; a saturated cyclic hydrocarbon group having 3 to 30, preferably 3 to 20 carbon atoms such as cyclopropyl, cyclobutyl, cyclopentyl, cyclohexyl and adamantyl; an unsaturated cyclic hydrocarbon group having 5 to 30 carbon atoms such as cyclopentadienyl, indenyl and fluorenyl; an aryl group having 6 to 30, preferably 6 to 20 carbon atoms such as phenyl, benzyl, naphthyl, biphenyl, terphenyl, phenanthryl and anthracenyl; and an alkyl-substituted aryl group such as tolyl, isopropyl phenyl, t-butyl phenyl, dimethylphenyl and di-t-butyl phenyl.

In the hydrocarbon group, a hydrogen atom may be substituted by halogen, and examples of the hydrocarbon group include a halogenated hydrocarbon group having 1 to 30 and preferably 1 to 20 carbon atom(s) such as trifluoromethyl, pentafluorophenyl and chlorophenyl.

Additionally, in the hydrocarbon group, a hydrogen atom may be substituted by another hydrocarbon group, and examples of the hydrocarbon group include an aryl group-substituted alkyl group such as benzyl and cumyl.

In addition, the hydrocarbon group include a heterocyclic compound residue; an oxygen-containing group such as an alkoxy group, an aryloxy group, an ester group, an ether group, an acyl group, a carboxyl group, a carbonate group, a hydroxy group, a peroxy group and a carboxylic acid anhydride group; a nitrogen-containing group such as an amino group, an imino group, an amide group, an imide group, a hydrazino group, a hydrazono group, a nitro group, a nitroso group, a cyano group, an isocyano group, a cyanic acid ester group, an amidino group, a diazo group, and an amino group present in the form of an ammonium salt; a boron-containing group such as a boranediyl group, a boranetriyl group and a diboranyl group; a sulfur-containing group such as a mercapto group, a thioester group, a dithioester group, an alkylthio group, an arylthio group, a thioacyl group, a thioether group, a thiocyanic acid ester group, an isothiocyanic acid ester group, a sulfone ester group, a sulfone amide group, a thiocarboxyl group, a dithiocarboxyl group, a sulfo group, a sulfonyl group, a sulfinyl group and a sulphenyl group; a phosphorus-containing group such as a phosphido group, a phosphoryl group, a thiophosphoryl group and a phosphate group; a silicon-containing group, a germanium-containing group or a tin-containing group.

Particularly, it is preferable to use a straight-chained or branched alkyl group having 1 to 30, preferably 1 to 20, more preferably 1 to 10, and most preferably 2 to 10 carbon atom(s) such as methyl, ethyl, n-propyl, iso-propyl, n-butyl, iso-butyl, sec-butyl, t-butyl, neopentyl, n-hexyl and adamantyl; an aryl group having 6 to 30 and preferably 6 to 20 carbon atoms such as phenyl, naphthyl, biphenyl, terphenyl, phenanthryl and anthracenyl; or a substituted aryl group which is substituted by 1 to 5 substituent(s) such as a halogen atom, an alkyl group or an alkoxy group having 1 to 30 and preferably 1 to 20 carbon atom(s), or an aryl group or an aryloxy group having 6 to 30 and preferably 6 to 20 carbon atoms.

Examples of the heterocyclic compound residue include residues of a nitrogen-containing compound such as pyrrole, pyridine, pyrimidine, quinoline, and triazine, an oxygen-containing compound such as furan and pyrane, a sulfur-containing compound such as thiophene; and groups in which a substituent such as an alkyl group or an alkoxy group having 1 to 30, preferably 1 to 20 carbon atom(s) is substituted to the heterocyclic compound residues thereof.

As the oxygen-containing group, the nitrogen-containing group, the sulfur-containing group, and the phosphorus-containing group, which are represented by $R^1$ to $R^{10}$, same ones mentioned as the substituent, which may be included in the hydrocarbon group, can be mentioned.

As the boron-containing group, groups such as alkyl group-substituted boron, aryl group-substituted boron, halogenated boron and alkyl group-substituted halogenated boron can be mentioned in addition to the same groups mentioned as the substituents which may be included in the hydrocarbon group. Examples of the alkyl group-substituted boron include $(Et)_2B$-, $(iPr)_2B$-, $(iBu)_2B$-, $(Et)_3B$, $(iPr)_3B$ and $(iBu)_3B$, examples of the aryl group-substituted boron include $(C_6H_5)_2B$-, $(C_6H_5)_3B$, $(C_6F_5)_3B$ and $(3,5\text{-}(CF_3)_2C_6H_3)_3B$, examples of the halogenated boron include $BCl_2$- and $BCl_3$, and examples of the alkyl group-substituted halogenated boron include (Et)BCl, (iBu)BCl- and $(C_6H_5)_2BCl$. Among them, trisubstituted boron may be in the linked state by a coordinate bond. Here, Et represents an ethyl group, iPr represents an iso-propyl group, and iBu represents an isobutyl group.

Examples of the aluminum-containing group include groups of alkyl group-substituted aluminum, aryl group-substituted aluminum, halogenated aluminum, and alkyl group-substituted halogenated aluminum. Examples of the alkyl group-substituted aluminum include $(Et)_2Al$-, $(iPr)_2Al$-, $(iBu)_2Al$-, $(Et)_3Al$, $(iPr)_3Al$, and $(iBu)_3Al$, examples of the aryl group-substituted aluminum include $(C_6H_5)_2Al$-, examples of the halogenated aluminum include $AlCl_2$- and $AlCl_3$, and examples of the alkyl group-substituted halogenated aluminum include (Et)AlCl- and (iBu)AlCl-. Among them, trisubstituted aluminum may be in the linked state by a coordinate bond. In this connection, Et represents an ethyl group, iPr represents an iso-propyl group, and iBu represents an iso-butyl group.

Examples of the silicon-containing group include a silyl group, a siloxy group, a hydrocarbon-substituted silyl group and a hydrocarbon-substituted siloxy group. Specific examples of the hydrocarbon-substituted silyl group include methylsilyl, dimethylsilyl, trimethylsilyl, ethylsilyl, diethylsilyl, triethylsilyl, diphenylmethylsilyl, triphenylsilyl, dimethylphenylsilyl, dimethyl-t-butylsilyl, and dimethyl(pentafluorophenyl)silyl. Among them, methylsilyl, dimethylsilyl, trimethylsilyl, ethylsilyl, diethylsilyl, triethylsilyl, dimethylphenylsilyl and triphenylsilyl are preferable. Trimethylsilyl, triethylsilyl, triphenylsilyl and dimethylphenylsilyl are more preferable. Specific examples of the hydrocarbon-substituted siloxy group include trimethylsiloxy.

Examples of the germanium-containing group and the tin-containing group include groups in which silicon of the silicon-containing group is substituted by germanium or tin. Among the above-mentioned nitrogen-containing groups, examples of the amide group include acetamide, N-methylacetamide and N-methylbenzamide, examples of the amino group include dimethylamino, ethylmethylamino and diphenylamino, examples of the imide group include acetimide and benzimide, and examples of the imino group include methylimino, ethylimino, propylimino, butylimino and phenylimino.

Among the above-mentioned sulfur-containing groups, examples of the alkylthio group include methylthio and ethylthio, examples of the arylthio group include phenylthio, methylphenylthio and naphthylthio, examples of the thioester group include acetylthio, benzoylthio, methylthiocarbonyl and phenylthiocarbonyl, examples of the sulfone ester group include sulfonic acid methyl, sulfonic acid ethyl and sulfonic acid phenyl, and examples of the sulfonamide group include phenyl sulfoneamide, N-methyl sulfoneamide and N-methyl-p-toluenesulfonamide.

In the $R^1$ to $R^{10}$, two or more groups and preferably adjacent groups may be bonded to each other to form aliphatic rings, aromatic rings or hydrocarbon rings containing heteroatoms such as nitrogen atoms, and the rings may further have substituent groups.
In the general formulae (I) and (II), M1 is a transition metal atom of Groups III to XI of the periodic table other than lanthanoid atoms and actinoid atoms, which is specifically, zirconium, titanium, hafnium, vanadium, niobium, tantalum, chromium, molybdenum or tungsten, preferably, zirconium, titanium, or hafnium, more preferably, zirconium or hafnium, and most preferably hafnium. Furthermore, n represents a valence of M1.

In the general formulae (I) and (II), X represents a hydrogen atom, a halogen atom, a hydrocarbon group, an oxygen-containing group, a sulfur-containing group, a nitrogen-containing group, a boron-containing group, an aluminum-containing group, a phosphorus-containing group, a halogen-containing group, a heterocyclic compound residue, a silicon-containing group, a germanium-containing group or a tin-containing group.
In connection with this, examples of the halogen atom include fluorine, chlorine, bromine and iodine.

Examples of the hydrocarbon group may be the same as those mentioned for the $R^1$ to $R^{10}$ of the general formula (I). Specific examples of the hydrocarbon group include, but are not limited to, an alkyl group such as methyl, ethyl, propyl, butyl, hexyl, octyl, nonyl, dodecyl and eicosyl; a cycloalkyl group having 3 to 30 carbon atoms such as cyclopentyl, cyclohexyl, norbornyl and adamantyl; an alkenyl group such as vinyl, propenyl and cyclohexenyl; an arylalkyl group such as benzyl, phenylethyl and phenylpropyl; and an aryl group such as phenyl, tolyl, dimethylphenyl, trimethylphenyl, ethylphenyl, propylphenyl, biphenyl, naphthyl, methylnaphthyl, anthryl and phenanthryl. Examples of the hydrocarbon group include halogenated hydrocarbon, in specific, a group in which at least one hydrogen of the hydrocarbon groups having 1 to 30 carbon atom(s) is substituted by halogen. Among them, hydrocarbon groups having 1 to 20 carbon atom(s) are preferable.

Additionally, examples of the heterocyclic compound residue may be the same as those mentioned for the $R^1$ to $R^{10}$ of the general formula (I).
Examples of the oxygen-containing group may be the same as those mentioned for the $R^1$ to $R^{10}$ of the general formula (I). Specific examples of the oxygen-containing group include, but are not limited to, a hydroxy group; an alkoxy group such as methoxy, ethoxy, propoxy, and butoxy; an aryloxy group such as phenoxy, methylphenoxy, dimethylphenoxy, and naphthoxy; an arylalkoxy group such as phenylmethoxy and phenylethoxy; an acetoxy group; and a carbonyl group.

Examples of the sulfur-containing group may be the same as those mentioned for $R^1$ to $R^{10}$ of the general formula (I). Specific examples of the sulfur-containing group include, but are not limited to a sulfonate group such as methylsulfonate, trifluoromethanesulfonate, phenylsulfonate, benzylsulfonate, p-toluenesulfonate, trimethylbenzenesulfonate, triisobutylbenzenesulfonate, p-chlorobenzenesulfonate and pentafluorobenzenesulfonate; a sulfinate group such as methylsulfinate, phenylsulfinate, benzylsulfinate, p-toluenesulfinate, trimethylbenzenesulfinate and pentafluorobenzenesulfinate; an alkylthio group; and an arylthio group.

Examples of the nitrogen-containing group may be the same as those mentioned for the $R^1$ to $R^{10}$ of the general formula (I). Specific examples of the nitrogen-containing group include, but are not limited to, an amino group; an alkylamino group such as methylamino, dimethylamino, diethylamino, dipropylamino, dibutylamino and dicyclohexylamino; and an arylamino group or an alkylarylamino group such as phenylamino, diphenylamino, ditolylamino, dinaphthylamino and methylphenylamino.

Specific examples of the boron-containing group include $BR_4$ (R represents hydrogen, an alkyl group, an aryl group which may have a substituent group, or a halogen atom etc) other than tetraphenyl borate.
Specific examples of the phosphorus-containing group include, but are not limited to, a trialkylphosphine group such as trimethylphosphine, tributylphosphine and tricyclohexylphosphine; a triarylphosphine group such as triphenylphosphine and tritolylphosphine; a phosphite group (phosphido group) such as methyl phosphate, ethyl phosphate and phenyl phosphate; a phosphonic acid group; and a phosphinic acid group.

Specific examples of the silicon-containing group may be the same as those mentioned for the $R^1$ to $R^{10}$ of the general formula (I). Specific examples of the silicon-containing group include a hydrocarbon-substituted silyl group such as phenylsilyl, diphenylsilyl, trimethylsilyl, triethylsilyl, tripropylsilyl, tricyclohexylsilyl, triphenylsilyl, methyldiphenylsilyl, tritolylsilyl and trinaphthylsilyl; a hydrocarbon-substituted silyl ether group such as trimethylsilyl ether; a silicon-substituted alkyl group such as trimethylsilyl methyl; and a silicon-substituted aryl group such as trimethylsilyl phenyl.

Specific examples of the germanium-containing group may be the same as those mentioned for the $R^1$ to $R^{10}$ of the general formula (I). Specific examples of the germanium-containing group include a group in which silicon of the silicon-containing group is substituted by germanium.
Specific examples of the tin-containing group may be the same as those mentioned for the $R^1$ to $R^{10}$ of the general formula (I). Specific examples of the tin-containing group include a group in which-silicon of the silicon-containing group is substituted by tin.

Specific examples of the halogen-containing group include, but are not limited to a fluorine-containing group such as $PF_6$ and $BF_4$, a chlorine-containing group such as $ClO_4$ and $SbCl_6$, and an iodine-containing group such as $IO_4$.
Specific examples of the aluminum-containing group include, but are not limited to, $AlR_4$ (R represents hydrogen, an

alkyl group, an aryl group which may have a substituent group, or a halogen atom).

Among them, the halogen atom and the alkyl group are preferable, and chlorine, bromine, and a methyl group are more preferable.

A synthesis of [A] the transition metal complexes which are represented by the general formulae (I) and (II) may be performed according to the processes disclosed in Organometallics, 1985, Vol. 4, pp. 97 - 104 and Macromolecules, 2000, Vol. 33, pp. 754 - 759.

After the reaction, the reaction product may be used directly as a mixture without being subjected to a purification process or may be used after being purified by a purification process such as recrystallization.

Next, the compounds of the component [B] will be described. The component [B] is at least one compound that is selected from the group consisting of (b-1) an organometallic compound, (b-2) an organoaluminumoxy compound, and (b-3) a compound which reacts with the transition metal complexes [A] to form an ion pair.

In the case where the transition metal complex [A1] represented by the general formula (I) is used as the component [A], the component [B] is employed if necessary. However, in the case where the transition metal complex [A2] represented by the general formula (II) is used as the component [A], it is essential to employ the component [B]. The component [B2] is at least one compound that is selected from the group consisting of (b2-1) an organometallic compound and (b2-3) a compound which reacts with the transition metal complex [A2] to form an ion pair.

The component [B2] when the component [A2] is employed may be exemplified by only the component (b2-1), only the component (b2-3), the components (b2-1) and (b2-3), or these components added with the component (b2-2). Hereinafter, the component (b-1), the component (b-2), and the component (b-3) will be described.

[(b-1) Organometallic Compound]

The organometallic compound (b-1) used in the invention may be specifically exemplified by the following organometallic compounds of Groups I, II, XII and XIII of the periodic table, which will be explained below. In the invention, the organometallic compound (b-1) of the compound [B] does not include the organoaluminumoxy compound (b-2) to be described later.

(b-1a) organoaluminum compound that is represented by a general formula $R^a{}_mAl(OR^b)_nH_pX_q$ (where, $R^a$ and $R^b$, which may be the same with or different from each other, are each a hydrocarbon group having 1 to 15 and preferably 1 to 4 carbon atom(s), X represents a halogen atom, m is a number satisfying $0 < m \leq 3$, n is a number satisfying $0 \leq n < 3$, p is a number satisfying $0 \leq p < 3$, and q is a number satisfying $0 \leq q < 3$, while $m + n + p + q = 3$).

(b-1b) alkylated complex of Group I metal of the periodic table and aluminum, which is represented by a general formula $M^2AlR^a{}_4$ (wherein, $M^2$ represents Li, Na, or K, and $R^a$ represents a hydrocarbon group having 1 to 15 and preferably 1 to 4 carbon atom(s)).

(b-1c) dialkyl compound of Group II or XII metal of the periodic table, which is represented by a general formula $R^aR^bM^3$ (wherein, $R^a$ and $R^b$ represent each a hydrocarbon group having 1 to 15 and preferably 1 to 4 carbon atom(s) which may or may not be the same, and $M^3$ represents Mg, Zn, or Cd).

Examples of the organoaluminum compound that belongs to (b-1a) include the following compounds: an organoaluminum compound that is represented by a general formula $R^a{}_mAl(OR^b)_{3-m}$ (wherein, $R^a$ and $R^b$ represent each a hydrocarbon group having 1 to 15 and preferably 1 to 4 carbon atom(s) which may or may not be the same, and m is preferably a number satisfying $1.5 \leq m \leq 3$), an organoaluminum compound that is represented by a general formula $R^a{}_mAlX_{3-m}$ (wherein, $R^a$ represents a hydrocarbon group having 1 to 15 and preferably 1 to 4 carbon atom(s), X represents a halogen atom, and m is preferably a number satisfying $0 < m < 3$), an organoaluminum compound that is represented by a general formula $R^a{}_mAlH_{3-m}$ (wherein, $R^a$ represents a hydrocarbon group having 1 to 15 and preferably 1 to 4 carbon atom(s), and m is preferably a number satisfying $2 \leq m < 3$), and an organoaluminum compound that is represented by a general formula $R^a{}_mAl(OR^b)_nX_q$ (wherein, $R^a$ and $R^b$ represent each a hydrocarbon group having 1 to 15 and preferably 1 to 4 carbon atom(s) which may or may not be the same, X represents a halogen atom, m is a number satisfying $0 < m \leq 3$, n is a number satisfying $0 \leq n < 33$, and q is a number satisfying $0 \leq q < 3$, while $m + n + q = 3$).

Specific examples of the organoaluminum compound that belongs to (b-1a) include tri(n-alkyl)aluminum such as trimethylaluminum, triethylaluminum, tri(n-butyl)aluminum, tripropylaluminum, tripentylaluminum, trihexylaluminum, trioctylaluminum and tridecylaluminum; tri branched-chain alkyl aluminum such as triisopropylaluminum, triisobutylaluminum, tri(sec-butyl)aluminum, tri(tert-butyl) aluminum, tri(2-methylbutyl)aluminum, tri(3-methylbutyl) aluminum, tri (2-methylpentyl)aluminum, tri (3-methylpentyl) aluminum, tri(4-methylpentyl)aluminum, tri(2-methylhexyl)aluminum, tri(3-methylhexyl)aluminum and tri(2-ethylhexyl)alumirium; tricycloalkylaluminum such as tricyclohexylaluminum and tricyclooctylaluminum; triarylaluminum such as triphenylaluminum and tritolylaluminum; dialkylaluminum hydride such as diethylaluminum hydride and diisobutylaluminum hydride; alkenylaluminum such as isoprenylaluminum that is represented by $(iC_4H_9)_xAl_y(C_5H_{10})_2$ (wherein, x, y, and z are each an integer while satisfying $z \geq 2x$, and $iC_4H_9$ represents an isobutyl group); alkylaluminum alkoxide such as isobutylaluminum methoxide, isobutylaluminum ethoxide and isobutylaluminum isopropoxide; dialkylaluminum alkoxide such as dimethylaluminum methoxide, diethylaluminum ethoxide

and dibutylaluminum butoxide; alkylaluminum sesquialkoxide such as ethylaluminum sesquiethoxide and butylaluminum sesquibutoxide; partially alkoxylated alkylaluminum that has an average composition represented by $R^a_{2.5}Al(OR^b)_{0.5}$ (wherein, $R^a$ and $R^b$ are each a hydrocarbon group having 1 to 15 and preferably 1 to 4 carbon atom(s) which may or may not be the same); dialkylaluminum aryloxide such as diethylaluminum phenoxide, diethyl aluminum(2,6-di-t-butyl-4-methylphenoxide), ethylaluminum bis(2,6-di-t-butyl-4-methylphenoxide), diisobutylaluminum(2,6-di-t-butyl-4-methylphenoxide) and isobutylaluminum bis(2,6-di-t-butyl-4-methylphenoxide); dialkylaluminum halide such as dimethylaluminum chloride, diethylaluminum chloride, dibutylaluminum chloride, diethylaluminum bromide and diisobutylaluminum chloride; alkylaluminum sesquihalide such as ethylaluminum sesquichloride, butylaluminum sesquichloride and ethylaluminum sesquibromide; partially halogenated alkylaluminum such as alkylaluminum dihalide including ethylaluminum dichloride, propylaluminum dichloride, and butylaluminum dibromide; dialkylaluminum hydride such as diethylaluminum hydride and dibutylaluminum hydride; alkylaluminum such as alkylaluminum dihydride including ethylaluminum dihydride and propylaluminum dihydride which is partially hydrogenated; and partially alkoxylated and halogenated alkylaluminum such as ethylaluminum ethoxychloride, butylaluminum buthoxychloride and ethylaluminum ethoxybromide.

Furthermore, a compound that is similar to the (b-1a) may be used. For example, an organoaluminum compound in which two or more aluminum compounds are bonded to each other through nitrogen atoms may be used. Specific examples of the compound include $(C_2H_5)_2AlN(C_2H_5)Al(C_2H_5)_2$. Examples of the compound that belongs to the (b-1b) include $LiAl(C_2H_5)_4$ and $LiAl(C_7H_{15})_4$.

Furthermore, examples of the organometallic compound (b-1) including methyllithium, ethyllithium, propyllithium, butyllithium, methylmagnesium bromide, methylmagnesium chloride, ethylmagnesium bromide, ethylmagnesium chloride, propylmagnesium bromide, propylmagnesium chloride, butylmagnesium bromide, butylmagnesium chloride, dimethylmagnesium, diethylmagnesium, dibutylmagnesium and butylethylmagnesium can also be used.

In addition, the same compound as the organoaluminum compound which is formed in a dimerization system, for example, a combination of halogenated aluminum and alkyllithium or a combination of halogenated aluminum and alkyl magnesium, may be used.

Among the organometallic compounds (b-1), the organoaluminum compound is preferable. The organometallic compounds (b-1) may be used singly or in combination of two or more kinds.

[(b-2) Organoaluminumoxy Compound]

In the invention, the organoaluminumoxy compound (b-2), which is used if necessary, may be known aluminoxane or a benzene insoluble organoaluminumoxy compound disclosed in JP-A-1990-78687. Known aluminoxane may be produced by using the following process normally as a solution containing a hydrocarbon solvent: (1) A process in which an organoaluminum compound such as trialkylaluminum is added to a hydrocarbon medium suspension liquid of a compound that contains adsorption water or salts that contain crystallization water, for example, such as magnesium chloride hydrate, cupric sulfate hydrate, aluminum sulfate hydrate, nickel sulfate hydrate and cerium chloride hydrate, and the adsorption water or the crystallization water is reacted with the organoaluminum compound; (2) A process in which water, ice, or steam is directly applied to an organoaluminum compound such as trialkylaluminum in a medium such as benzene, toluene, ethyl ether and tetrahydrofuran; and (3) A process in which organic tin oxides such as dimethyltin oxides and dibutyltin oxides are reacted with an organoaluminum compound such as trialkylaluminum in a medium such as decane, benzene and toluene.

The aluminoxane may contain a small amount of organic metal component. Alternatively, after the solvent or the unreacted organoaluminum compound is distilled off from the recovered aluminoxane solution, the resulting solute may be resolved in a solvent or suspended in poor solvent of aluminoxane. Specific examples of the organoaluminum compound that is used to produce aluminoxane include the same organoaluminum compounds as the organoaluminum compounds belonging to the (b-1a).

Among them, trialkylaluminum and tricycloalkylaluminum are preferable, and trimethylaluminum is more preferable.

The organoaluminum compound may be used singly or in combination of two or more kinds.

Examples of the solvent that is used to produce aluminoxane include hydrocarbon solvents including aromatic hydrocarbons such as benzene, toluene, xylene, cumene and cymene; aliphatic hydrocarbons such as pentane, hexane, heptane, octane, decane, dodecane, hexadecane and octadecane; alicyclic hydrocarbons such as cyclopentane, cyclohexane, cyclooctane and methylcyclopentane; petroleum fractions such as gasoline, kerosene and diesel; or halogenates of the aromatic hydrocarbons, the aliphatic hydrocarbons and the alicyclic hydrocarbons, particularly, chlorinates or brominates thereof. Furthermore, ethers such as ethyl ether and tetrahydrofuran may be mentioned. Among the above-mentioned solvents, the aromatic hydrocarbons or the aliphatic hydrocarbons are preferable.

As to the benzene insoluble organoaluminumoxy compound used in the invention, it is required that the amount of the Al component that is solved in benzene at 60°C is 10% or less, preferably 5% or less, and more preferably 2% or less in terms of Al atom, that is, the organoaluminumoxy compound that is insoluble or slightly soluble in benzene is preferable.

Examples of the organoaluminumoxy compound, which is used in the invention, include an organoaluminumoxy compound containing boron that is represented by the following general formula (i).

[Chem. 5]

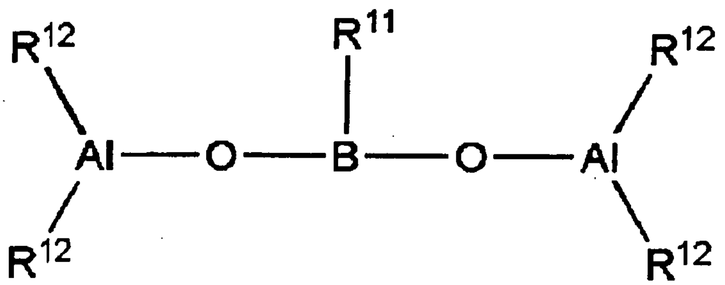

(i)

wherein, $R^{11}$ represents a hydrocarbon group having 1 to 10 carbon atom(s). $R^{12}$ represents a hydrogen atom, a halogen atom or a hydrocarbon group having 1 to 10 carbon atom(s), which may or may not be the same.
The organoaluminumoxy compound containing boron that is represented by the general formula (i) may be produced by reacting an alkylboronic acid represented by the following general formula (ii) and an organoaluminum compound in an inert solvent under inert gas atmosphere at a temperature of ranging -80°C to room temperature for 1 min to 24 hours.
$R^{11}$-B $(OH)_2$ (ii)
wherein, $R^{11}$ is as described above.

Specific examples of the alkylboronic acid represented by the general formula (ii) include a methylboronic acid, an ethyl boronic acid, an isopropylboronic acid, a n-propylboronic acid, a n-butylboronic acid, an isobutylboronic acid, a n-hexylboronic acid, a cyclohexylboronic acid, a phenylboronic acid, a 3,5-difluorophenylboronic acid, a pentafluorophenylboronic acid and a 3,5-bis(trifluoromethyl)phenylboronic acid. Among them, methylboronic acid, n-butylboronic acid, isobutylboronic acid, 3,5-difluorophenylboronic acid and pentafluorophenylboronic acid are preferable.

They are used singly or in combination of two or more kinds.
Specifically, the organoaluminum compound that is reacted with the alkylboronic acid may be the same organoaluminum compound as the organoaluminum compound belonging to the (b-1a). Among them, trialkylaluminum and tricycloalkylaluminum are preferable, and trimethylaluminum, triethylaluminum and tri-isobutyl aluminum are more preferable. They are used singly or in combination of two or more kinds.

The organoaluminumoxy compounds (b-2) may be used singly or in combination of two or more kinds.
However, in the case where the transition metal complex [A] is decamethylhafnocene dichloride or decamethylzirconocene dichloride, the organoaluminumoxy compound (b-2) cannot be used.

[(b-3) Ionized Ionic Compound]
In the invention, the compound (b-3) which reacts with the transition metal complex [A] used if necessary to form an ion pair is a compound which reacts with the transition metal complex [A] to form an ion pair. Accordingly, this compound contains any compound as long as the compound comes into contact with the transition metal complex [A] to form an ion pair.

Examples of the compound include a Lewis acid, an ionic compound, a borane compound and a carborane compound that are disclosed in JP-A-1989-501950, JP-A-1989-502036, JP-A-1991-179005, JP-A-1991-179006, JP-A-1991-207703, JP-A-1991-307704 and US Pat. No. 5,321,106. Furthermore, a heteropoly compound and an isopoly compound may be mentioned.

Specifically, examples of the Lewis acid include a compound that is represented by $BR_3$ (R represents fluorine or a phenyl group which may have a substituent group such as fluorine, a methyl group, or a trifluoromethyl group). Examples of the compound include trifluoroboron, triphenylboron, tris (4-fluorophenyl)boron, tris(3,5-difluorophenyl) boron, tris (4-fluoromethylphenyl)boron, tris(pentafluorophenyl)boron, tris(p-tolyl)boron, tris(o-tolyl)boron, and tris(3,5-dimethylphenyl)boron.

Examples of the ionic compound include a compound represented by the following general formula (III).

[Chem. 6]

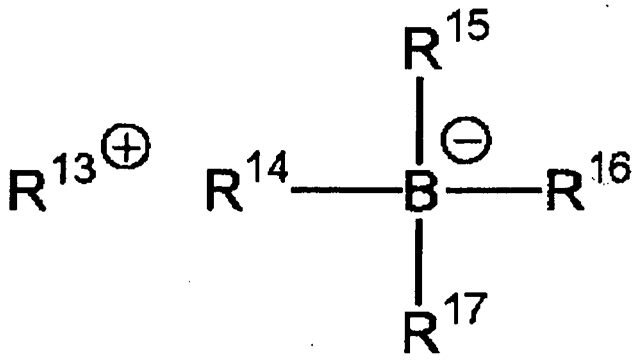

(III)

wherein, $R^{13+}$ may be exemplified by $H^+$, a carbonium cation, an oxonium cation, an ammonium cation, a phosphonium cation, a cycloheptyltrienyl cation or a ferrocenium cation having a transition metal.
Each of $R^{13}$ to $R^{17}$ is an organic group, and preferably an aryl group or a substituted aryl group, which may or may not be the same.

Specific examples of the carbonium cation include 3-substituted carbonium cation such as a triphenylcarbonium cation, a tri(methylphenyl)carbonium cation and a tri(dimethylphenyl)carbonium cation.
Specific examples of the ammonium cation include a trialkylammonium cation such as a trimethylammonium cation, a triethylammonium cation, a tri(n-propyl)ammonium cation and a tri(n-butyl)ammonium cation; an N, N-dialkylanilinium cation such as an N, N-dimethylanilinium cation, an N, N-diethylanilinium cation and an N, N, 2, 4, 6-pentamethylanilinium cation; and a dialkylammonium cation such as a di(isopropyl)ammonium cation and a dicyclohexylammonium cation.

Specific examples of the phosphonium cation include a triarylphosphonium cation such as a triphenylphosphonium cation, a tri(methylphenyl)phosphonium cation and a tri(dimethylphenyl)phosphonium cation.
$R^{13+}$ is preferably a carbonium cation or an ammonium cation, and more preferably a triphenylcarbonium cation, an N, N-dimethylanilinium cation or an N, N-diethylanilinium cation.

Furthermore, examples of the ionic compound include trialkyl-substituted ammonium salts, N, N-dialkylanilinium salts, dialkylammonium salts and triarylphosphonium salts.
Specific examples of the trialkyl-substituted ammonium salts include triethylammonium tetraphenylborate, tri(n-propyl) ammonium tetraphenylborate, tri(n-butyl)ammonium tetraphenylborate, trimethylammonium tetra(p-tolyl)borate, trimethylammonium tetra (o-tolyl) borate, tri(n-butyl)ammonium tetra(pentafluorophenyl)borate, tri(n-propyl)ammonium tetra(o, p-dimethylphenyl)borate, tri(n-butyl)ammonium tetra(m, m-dimethylphenyl)borate, tri(n-butyl)ammonium tetra(p-trifluoromethylphenyl)borate, tri(n-butyl)ammonium tetra(3,5-ditrifluoromethylphenyl)borate and tri(n-butyl)ammonium tetra(o-tolyl)borate.

Specific examples of the N, N-dialkylanilinium salts include N, N-dimethylanilinium tetraphenylborate, N, N-diethylanilinium tetraphenylborate and N, N, 2, 4, 6-pentamethylanilinium tetraphenylborate.
Specific examples of the dialkylammonium salts include di(n-propyl)ammonium tetra(pentafluorophenyl)borate and dicyclohexylammonium tetraphenylborate.

Furthermore, examples of the ionic compound include triphenylcarbenium tetrakis(pentafluorophenyl)borate, N, N-dimethylanilinium tetrakis(pentafluorophenyl)borate, ferrocenium tetra(pentafluorophenyl)borate, a triphenylcarbeniumpentaphenylcyclopentadienyl complex, an N, N-diethylaniliniumpentaphenylcyclopentadienyl complex or a boron compound represented by the following formula (IV) or (V) .

[Chem.7]

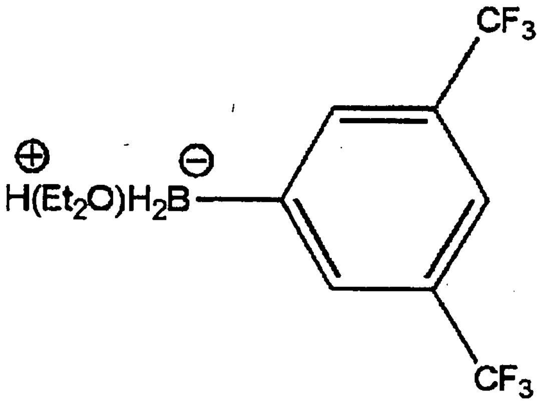

(IV)

(wherein, Et represents an ethyl group.)

[Chem. 8]

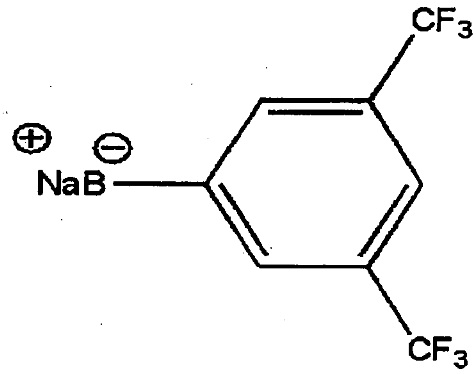

(V)

Specific examples of the boran compound include decaborane (14); anion salts such as bis [tri(n-butyl)ammonium] nonaborate, bis [tri(n-butyl)ammonium] decaborate, bis [tri(n-butyl)ammonium] undecaborate, bis [tri(n-butyl)ammonium] dodecaborate, bis [tri(n-butyl)ammonium] decachlorodecaborate and bis [tri(n-butyl)ammonium) dodecachlorododecaborate; and metal boran anion salts such as tri(n-butyl)ammonium bis(dodecahydridedodecaborate)cobaltates (III) and bis [tri(n-butyl)ammonium] bis (dodecahydridedodecaborate) nickelates (III) .

Specific examples of the carborane compound include anion salts such as 4-carbanonaborane (14), 1,3-dicarbanonaborane (13), 6,9-dicarbadecaborane (14), dodecahydride-1-phenyl-1,3-dicarbanonaborane, dodecahydride-1-methyl-1,3-dicarbanonaborane, undecahydride-1,3-dimethyl-1,3-dicarbanonaborane, 7,8-dicarbaundecaborane (13), 2,7-dicarbaundecaborane (13), undecahydride-7,8-dimethyl-7,8-dicarbaundecaborane, dodecahydride-11-methyl-2,7-dicarbaundecaborane, tri(n-butyl)ammonium 1 carbadecaborate, tri(n-butyl)ammonium 1-carbaundecaborate, tri(n-butyl)ammonium 1-carbadodecaborate, tri(n-butyl)ammonium 1-trimethylsilyl-1-carbadecaborate, tri(n-butyl)ammonium bromo-1-carbadodecaborate, tri(n-butyl)ammonium 6-carbadecaborate (14), tri(n-butyl)ammonium 6-carbadecaborate (12), tri(n-butyl)ammonium 7 carbaundecaborate (13), tri(n-butyl)ammonium 7,8-dicarbaundecaborate (12), tri(n-butyl) ammonium 2,9-dicarbaundecaborate (12), tri(n-butyl)ammonium dodecahydride-8-methyl-7,9-dicarbaundecaborate, tri (n-butyl)ammonium undecahydride-8-ethyl-7,9-dicarbaundecaborate, tri(n-butyl)ammonium undecahydride-8-butyl-7,9-dicarbaundecaborate, tri(n-butyl)ammonium undecahydride-8-aryl-7,9-dicarbaundecaborate, tri(n-butyl)ammonium undecahydride-9-trimethylsilyl-7,8-dicarbaundecaborate and tri(n-butyl)ammonium undecahydride-4,6-dibromo-7-carbaundecaborate; and metal carborane anion salts such as tri(n-butyl)ammonium bis(nonahydride-1,3-dicarbanonaborate)cobaltates (III), tri(n-butyl)ammonium bis(undecahydride-7,8-dicarbaundecaborate)ferrates (III), tri(n-butyl)ammonium bis(undecahydride-7,8-dicarbaundecaborate)cobaltates (III), tri(n-butyl)ammonium bis(undecahydride-7,8-dicarbaundecaborate)nickelates (III), tri(n-butyl)ammonium bis(undecahydride-7,8-dicarbaundecaborate)cuprates (III), tri(n-butyl)ammonium bis(undecahydride-7,8-dicarbaundecaborate)aurates (III), tri(n-butyl)ammonium bis(nonahydride-7,8-dimethyl-7,8-dicarbaundecaborate)ferrates (III), tri(n-butyl)ammonium bis(nonahydride-7,8-dimethyl-7,8-dicarbaundecaborate)chromates (III), tri(n-butyl)ammonium bis(tribromooctahydride-7,8-dicarbaundecaborate)cobaltates (III), tris

[tri(n-butyl)ammonium] bis (undecahydride-7-carbaundecaborate)chromates (III), bis [tri(n-butyl) ammonium] bis(undecahydride-7-carbaundecaborate)manganates (IV), bis [tri(n-butyl) ammonium] bis(undecahydride-7-carbaundecaborate) cobaltates (III) and bis [tri(n-butyl)ammonium] bis(undecahydride-7-carbaundecaborate)nickelates (IV).

The heteropoly compound contains an atom of silicon, phosphorus, titanium, germanium, arsenic or tin, and one or more atoms selected from vanadium, niobium, molybdenum and tungsten. Specific examples of the heteropoly compound include, but are not limited to a phosphovanadic acid, a germanovanadic acid, an arsenovanadic acid, a phosphoniobic acid, a germanoniobic acid, a siliconomolybdic acid, a phosphomolybdic acid, a titanomolybdic acid, a germanomolybdic acid, an arsenomolybdic acid, a stannomolybdic acid, a phosphotungstic acid, a germanotungstic acid, a stannotungstic acid, a phosphomolybdovanadic acid, a phosphotungstovanadic acid, a germanotungstovanadic acid, a phosphomolybdotungstovanadic acid, a germanomolybdotungstovanadic acid, a phosphomolybdotungstic acid and a phosphomolybdoniobic acid, salts of these acids, for example, salts with metal of Group I or II of the periodic table, specifically, lithium, sodium, potassium, rubidium, cesium, beryllium, magnesium, calcium, strontium and barium, organic salts such as triphenylethyl salts, and an isopoly compound.

Not only one but also two or more kind of compounds may be used as the heteropoly compound and the isopoly compound.

The above-mentioned ionized ionic compounds (b-3) may be used singly or in combination of two or more kinds.

If a propylene dimerization catalyst of the invention is used, propylene dimers are obtained with high dimerization activity. Particularly, selectivity of 4-methyl-1-pentene is high. If the organoaluminumoxy compound (b-2) such as methyl aluminoxane is used at the same time as a cocatalyst component, very high dimerization activity is obtained in respect to the propylene compound. If the ionized ionic compound (B-3) such as triphenylcarbonium tetrakis(pentafluorophenyl)borate is used as the cocatalyst component, 4-methyl-1-pentene is obtained with good activity and very high selectivity.

Furthermore, the transition metal complex [A] and at least one compound [B] that is selected from (b-1) the organometallic compound, (b-2) the organoaluminumoxy compound, and (b-3) the ionized ionic compound may be used as the catalyst for propylene dimerization according to the invention, and a carrier [C] as described below may be additionally used if necessary.

[[C] Carrier]

The carrier [C], which is used if necessary in the invention, is an inorganic or organic compound, and a granule or particle type of solid. Among them, porous oxide, inorganic chloride, clay, clay mineral, or ion-exchangeable layered compounds are preferably used as the inorganic compound.

Specific examples of the porous oxide include $SiO_2$, $Al_2O_3$, MgO, ZrO, $TiO_2$, $B_2O_3$, CaO, ZnO, BaO, $ThO_2$ or a complex or mixture thereof, for example, natural or synthetic zeolite, $SiO_2$-MgO, $SiO_2$-$Al_2O_3$, $SiO_2$-$TiO_2$, $SiO_2$-$V_2O_5$, $SiO_2$-$Cr_2O_3$, and $SiO_2$-$TiO_2$-MgO. Among them, the porous oxides containing $SiO_2$ and/or $Al_2O_3$ as main components are preferably used.

Furthermore, there is no problem that the inorganic oxide may contain a small amount of carbonate, sulfate, nitrate and oxide components such as $Na_2CO_3$, $K_2CO_3$, $CaCO_3$, $MgCO_3$, $Na_2SO_4$, $Al_2(SO_4)_3$, $BaSO_4$, $KNO_3$, $Mg(NO_3)_2$, $Al(NO_3)_3$, $Na_2O$, $K_2O$ and $Li_2O$.

The properties of the porous oxide depend on the type and the prearation method thereof. The carrier that is used in the invention preferably has a particle size of 10 to 300 μm and more preferably 20 to 200 μm, a specific surface area of 50 to 1000 $m^2$/g and more preferably 100 to 700 $m^2$/g, and a pore volume of 0.3 to 3.0 $cm^3$/g. The carrier may be burned at 100 to 1000°C and preferably 150 to 700°C as required.

Examples of the inorganic chloride include $MgCl_2$, $MgBr_2$, $MnCl_2$ and $MnBr_2$. The inorganic chloride may be used as it is or may be used after pulverizing the inorganic chloride with a ball mill or a vibration mill. The inorganic chloride may be used as granules by disoving it in a solvent such as alcohol and adding an educing agent thereto to educe the inorganic chloride as a form of granules.

The clay, which is used as the carrier in the invention, generally contains clay minerals as main components.

Furthermore, the ion-exchangeable layered compound, which is used as the carrier in the invention, is a compound having a crystal structure wherein planes formed by ionic bonding etc. overlap parallel to each other through weak bonding force, and ions that are contained in the compound are capable of being exchanged. Most of clay minerals are ion-exchangeable layered compound. Furthermore, the clay, the clay mineral, and the ion-exchangeable layered compound are not limited to natural compounds but artificial synthetic compounds may be used.

Examples of the clay, the clay mineral, or the ion-exchangeable layered compound include the clay, the clay mineral or an ionic crystalline compound having a layered crystal structure such as a hexagonal close-packed type, an antimony type, a $CdCl_2$ type and a $CdI_2$ type.

Examples of the clay and the clay mineral include kaolin, bentonite, Kibushi clay, Potter's clay, allophane, hisingerite, pyrophyllite, micas, montmorillonites, vermiculite, chlorites, palygorskite, kaolinite, nakhlite, dickite and halloysite, and examples of the ion-exchangeable layered compound include crystalline acid salts of polyvalent metal such as $\alpha$-$Zr(HAsO_4)_2 \cdot H_2O$, $\alpha$-$Zr(KPO_4)_2 \cdot 3H_2O$, $\alpha$-$Ti(HPO_4)_2$, $\alpha$-$Ti(HAsO_4)_2 \cdot H_2O$, $\alpha$-$Sn(HPO_4)_2 \cdot H_2O$, $\gamma$-$Zr(HPO_4)_2$, $\gamma$-$Ti(HPO_4)_2$, and $\gamma$-$Ti(NH_4PO_4)_2 \cdot H_2O$.

The pore volume of the clay, clay mineral, or the ion-exchangeable layered compound that is measured by using mercury intrusion porosimetry in respect to pores having a radius of 20 angstroms or more is preferably 0.1 cc/g or more and more preferably 0.3 to 5 cc/g. In this connection, the pore volume is measured by means of the mercury intrusion porosimetry using a mercury porosimeter in respect to the pore radius in the range of 20 to $3 \times 10^4$ angstroms. In the case when the carrier having the pore volume of less than 0.1 cc/g in respect to the pores having the radius of 20 angstroms or more is used, high dimerization activity tends not to be ensured.

The clay and the clay mineral used in the invention may be subjected to chemical treatment. The chemical treatment may be any one of surface treatment of removing impurities attached to the surface and affecting a crystal structure of the clay. Specific examples of the chemical treatment include acid treatment, alkali treatment, treatment with salts, and treatment with organic compounds. The acid treatment works not only to remove impurities from the surface and but also to elute cations such as Al, Fe and Mg from a crystal structure, thereby increasing a surface area. In the alkali treatment, the crystal structure of the clay is destroyed, causing transformation of the clay structure. The treatment with salts and the treatment with organic compounds can cause a formation of ion complexes, molecule complexes, and organic derivatives and a change of the surface area or the distance between the layers.

The ion-exchangeable layered compound used in the invention may be a layered compound wherein the interval between the layers are changed to a enlarged state by replacing exchangeable ions between the layers with ions each having a large volume by means of the ion exchangeable property. Such bulky ions function to support the layer structure like a pillar; accordingly, they are called pillars. Furthermore, introducing other substances between the layers of the layered compound is called intercalation. Examples of the guest compound used in the intercalation include cationic inorganic compounds such as $TiCl_4$ and $ZrCl_4$; metal alkoxides such as $Ti(OR)_4$, $Zr(OR)_4$, $PO(OR)_3$ and $B(OR)_3$ (R is a hydrocarbon group, etc.); and metal hydroxide ions such as $[Al_{13}O_4(OH)_{24}]^{7+}$, $[Zr_4(OH)_{14}]^{2+}$ and $[Fe_3O(OCOCH_3)_6]^+$.

These compounds are used singly or in combination of two or more kinds.

In the case when the compounds are subjected to intercalation, dimers that are obtained by hydrolyzing metal alkoxides such as $Si(OR)_4$, $Al(OR)_3$ and $Ge(OR)_4$ (R represents a hydrocarbon group, etc.), and colloidal inorganic compounds such as $SiO_2$ may be coexisted. Furthermore, examples of the pillar include oxides that are formed by performing heat dehydration after the metal hydroxide ions are intercalated between the layers.

The clay, the clay mineral and the ion-exchangeable layered compound, which are used in the invention, may be used as it is or may be used after treatment such as ball milling or screening is performed. Furthermore, they may be used after water is added and adsorbed or after heat dehydration is performed. They may be used singly or in combination of two or more kinds.

Among them, preferably, the clay or the clay mineral is used, and, more preferably, montmorillonite, vermiculite, hectorite, taylorite and synthetic mica are used.

Examples of the organic compound include a granule type or particle type solid that has particle sizes in the range of 10 to 300 $\mu$m. Specific example of the organic compound include (co-)dimers such as ethylene, propylene, 1-butene and 4-methyl-1-pentene which contains $\alpha$-olefin having 2 to 14 carbon atoms as a main component, (co-)dimers which contain vinyl cyclohexane and styrene as a main component, and derivatives thereof.

The catalyst for propylene dimerization according to the invention include the transition metal complex [A], at least one compound [B], as needed, that is selected from (b-1) the organometallic compound, (b-2) the organoaluminumoxy compound, and (b-3) the ionized ionic compound, the carrier [C] as needed and further a predetermined organic compound [D] as described below as needed.

[[D] Organic Compound Component]

In the invention, the organic compound component [D] is used to improve dimerization performance, as needed. Examples of the organic compound include, but are not limited to alcohols, phenol compounds, carboxylic acids, phosphorus compounds and sulfonates.

Alcohols and phenol compounds that are represented by $R^{18}$-OH (wherein, $R^{18}$ represents a hydrocarbon group having 1 to 50 carbon atom(s) or a halogenated hydrocarbon group having 1 to 50 carbon atom(s)) are generally used, and alcohols in which $R^{18}$ is a halogenated hydrocarbon group are preferable.

Furthermore, the phenol compounds in which $\alpha,\alpha'$-positions of the hydroxide group are substituted by hydrocarbon having 1 to 20 carbon atom(s) is preferable.

The carboxylic acids that are represented by $R^{19}$-COOH are generally used. The $R^{19}$ represents a hydrocarbon group having 1 to 50 carbon atom(s) or a halogenated hydrocarbon group having 1 to 50 carbon atom(s). Particularly, it is preferable to use the halogenated hydrocarbon group having 1 to 50 carbon atom(s).

Preferable examples of the phosphorus compound include phosphoric acids having a P-O-H bond and phosphates and phosphin oxide compounds having a P-OR or P=O bond.

The sulfonates may be represented by the following general formula (VI).

[Chem. 9]

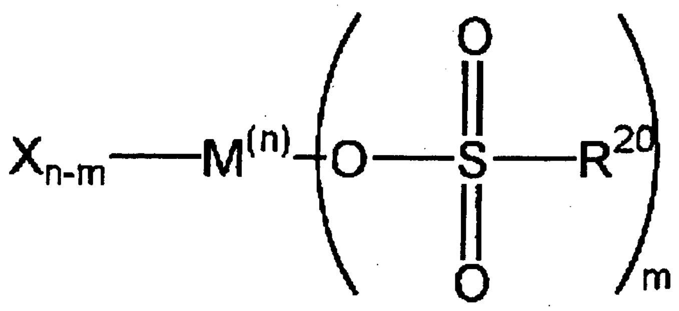

(VI)

wherein,, M is an element of Groups I to XIV of the periodic table. $R^{20}$ is hydrogen, a hydrocarbon group having 1 to 20 carbon atom(s) or a halogenated hydrocarbon group having 1 to 20 carbon atom(s). X is a hydrogen atom, a halogen atom, a hydrocarbon group having 1 to 20 carbon atom(s) or a halogenated hydrocarbon group having 1 to 20 carbon atom(s). M is an integer of 1 to 7, and n is a valence of M, where $1 \leq n \leq 7$.

[Method of Dimerizing Propylene]

Next, a method of dimerizing propylene will be described.

The process of dimerizing propylene according to the invention comprises dimerizing propylene in the presence of the catalyst.

During the dimerization, a process of adding the component [A] to the reactor, way of using the components, a process of adding each component and the order of addition thereof may be arbitrarily selected, and may be exemplified as follows.

(1) A process of adding only the component [A] to a reactor.

(2) A process of adding the component [A], and at least one component [B] selected from (b-1) the organometallic compound, (b-2) the organoaluminumoxy compound, and (b-3) the ionized ionic compound (hereinafter, referred to as "component [B]") to the reactor in the arbitrary order.

(3) A process of adding a catalyst in which the component [A] has been preliminarily brought into contact with the component [B], to the reactor.

(4) A process of adding a catalyst component in which the component [A] has been preliminarily brought into contact with the component [B], and component [B], to the reactor in the arbitrary order. In this case, the components [B] may or may not be the same.

(5) A process of adding only a catalyst component in which the component [A] is supported on the carrier [C], to the reactor.

(6) A process of adding a catalyst component in which the component [A] is supported on the carrier [C], and the component [B], to the reactor in the arbitrary order.

(7) A process of adding a catalyst in which the component [A] and the component [B] are supported on the carrier [C], to the reactor.

(8) A process of adding a catalyst component in which the component [A] and the component [B] are supported on the carrier [C], and the component [B], to the reactor in the arbitrary order. In this case, the components [B] may or may not be the same.

(9) A process of adding a catalyst component in which the component [B] is supported on the carrier [C], and the component [A], to the reactor in the arbitrary order.

(10) A process of adding a catalyst component in which the component [B] is supported on the carrier [C], the component [A], and the component [B], to the reactor in the arbitrary order. In this case, the components [B] may or may not be the same.

(11) A process of adding a component in which the component [A] is supported on the carrier [C], and a component in which the component [B] is supported on the carrier [C], to the reactor in the arbitrary order.

(12) A process of adding a component in which the component [A] is supported on the carrier [C], a component in which the component [B] is supported on the carrier [C], and the component [B], to the reactor in the arbitrary order. In this case, components [B] may or may not be the same.

(13) A process of adding the component [A] and the component [D] to the reactor in the arbitrary order.

(14) A process of adding the component [A], the component [B], and the organic compound component [D], to the

reactor in the arbitrary order.

(15) A process of adding a component in which the component [B] has been preliminarily brought into contact with the component [D], and the component [A], to the reactor in the arbitrary order.

(16) A process of adding a component in which the component [D] is supported on the carrier [C], and the component [A], to the reactor in the arbitrary order.

(17) A process of adding a component in which the component [B] and the component [D] are supported on the carrier [C], and the component [A], to the reactor in the arbitrary order.

(18) A process of adding a catalyst component in which the component [A] has been preliminarily brought into contact with the component [B], and the component [D], to the reactor in the arbitrary order.

(19) A process of adding a catalyst component in which the component [A] has been preliminarily brought into contact with the component [B], the component [B], and the component [D], to the reactor in the arbitrary order.

(20) A process of adding a catalyst component in which the component [A] has been preliminarily brought into contact with the component [B], and a component in which the component [B] has been preliminarily brought into contact with the component [D], to the reactor in the arbitrary order.

(21) A process of adding a component in which the component [A] is supported on the carrier [C], the component [B], and the component [D], to the reactor in the arbitrary order.

(22) A process of adding a component in which the component [A] is supported on the carrier [C], and the component [D], to the reactor in the arbitrary order.

(23) A process of adding a component in which the component [A] is supported on the carrier [C], and a component in which the component [B] has been preliminarily brought into contact with the component [D], to the reactor in the arbitrary order.

(24) A process of adding a catalyst component in which the component [A] has been preliminarily brought into contact with the component [D] to the reactor.

(25) A process of adding a catalyst component that has been formed by preliminarily contacting the component [A], the component [B], and the component [D] with each other in the arbitrary order, to the reactor.

(26) A process of adding a catalyst component in which the component [A], the component [B], and the component [D] have been preliminarily brought into contact with each other, and the component [B], to the reactor in the arbitrary order. In this case, the components [B] may or may not be the same.

(27) A process of adding a catalyst in which the component [A] and the component [D] are supported on the carrier [C], to the reactor.

(28) A process of adding a catalyst in which the component [A], the component [B], and the component [D] are supported on the carrier [C], to the reactor.

(29) A process of adding the catalyst component in which the component [A], the component [B], and the component [D] are supported on the carrier [C], and the component [B], to the reactor in the arbitrary order. In this case, the components [B] may or may not be the same.

In the method of producing 4-methyl-1-pentene according to the invention, propylene is dimerized in the presence of the above-mentioned propylene dimerization catalyst to produce 4-methyl-1-pentene. In the invention, the dimerization may be performed by using any one of a liquid phase reaction process such as a dissolution reaction or a suspension reaction and a gas phase reaction process.

Specific examples of the inert hydrocarbon medium that is used in the liquid phase dimerization process include aliphatic hydrocarbons such as propane, butane, pentane, hexane, heptane, octane, decane, dodecane and kerosen; alicyclic hydrocarbons such as cyclopentane, cyclohexane and methylcyclopentane; aromatic hydrocarbons such as benzene, toluene and xylene; halogenated hydrocarbons such as ethylene chloride, chlorobenzene, and dichloromethane; and mixtures thereof. Propylene may be used itself as the solvent.

In the case where 4-methyl-1-pentene is produced by using the above-mentioned catalyst for propylene dimerization, the component [A] is generally used in the amount that would be $10^{-12}$ to $10^{-2}$ mole, and preferably $10^{-10}$ to $10^{-3}$ mole, per 1 liter of the reaction volume. In the invention, even in a case where the component [A] is used at a relatively low concentration, propylene is capable of being dimerized with high dimerization activity.

Furthermore, in a case where the component [B] is used, the component (b-1) is generally used in an amount that a molar ratio of the component (b-1) and the transition metal complex atom (M1) of the component [A] [(b-1) / M1] would be in the range of from 0.01 to 100000, and preferably from 0.05 to 50000.

The component (b-2) is generally used in an amount that a molar ratio of the aluminum atom of the component (b-2) and the transition metal atom (M1) of the component [A] [(b-2) / M1] would be in the range of from 10 to 500000, and preferably from 20 to 100000.

The component (b-3) is generally used in an amount that a molar ratio of the component (b-3) and the transition metal atom (M1) of the component [A] [(b-3) / M1] would be in the range of from 1 to 10, and preferably from 1 to 5.

The component [D] is generally used in an amount that a molar ratio [(D) / (b-1)] would be in the range of from 0.01 to

10 and preferably from 0.1 to 5 in respect to the component (b-1) of the component [B], a molar ratio [(D) / (b-2)] of the component [D] and the aluminum atom of the component (b-2) would be in the range of from 0.001 to 2 and preferably from 0.005 to 1 in respect to the component (b-2) of the component [B], and a molar ratio [(D) / (b-3)] would be in the range of from 0.01 to 10, and preferably from 0.1 to 5 in respect to the component (b-3) of the component [B].

The temperature of the dimerization of propylene by using the propylene dimerization catalyst is in the range of generally from -50 to 200°C, and preferably from 0 to 170°C. The reaction pressure is generally from normal pressure to 100 $kg/cm^2$, and preferably from normal pressure to 50 $kg/cm^2$, and the dimerization reaction may be performed by using any one process of a batch process, a semicontinuous process, and a continuous process.

EXAMPLES

Hereinafter, the invention will be described in more detail with reference to Examples, but the invention is not limited by these Examples.
The yield of the reaction product and selectivity of 4-methyl-1-pentene were analyzed by using gas chromatography (Shimazu GC-2010, J&W Scientific DB-1 column).

[Catalytic activity]

The mass of the reaction product that is obtained per unit time was divided by the atomic weight (mmole) of the transition metal in the transition metal catalyst component used during polymerization to calculate the catalytic activity.

[Selectivity of 4-methyl-1-pentene]

The selectivity of 4-methyl-1-pentene was calculated by using the following equation.

$$S\ (\%) = Wp/Wr \times 100$$

S (%): selectivity of 4-methyl-1-pentene (weight fraction)
Wr (weight): Total weight of products having 4 or more carbon atoms that are produced by a reaction
Wp (weight): Weight of 4-methyl-1-pentene that is produced by the reaction
Specific Synthetic Examples of the transition metal compound according to the invention, and specific Examples and Comparative Examples of propylene dimerization will be described hereinafter.

[Synthetic Example 1]

1-ethyl-2,3,4,5-tetramethylcyclopentadienyllithium (107 mg), pentamethylcyclopentadienylhafnium trichloride (276 mg) and xylene (10 ml) were added to a reaction vessel and reacted therein under a reflux condition for 2 days. The solvent was distilled off under a reduced pressure, and dichloromethane (50 ml) and 1N-HCl aqueous solution (50 ml) were added to perform a liquid separation. The separated aqueous solution was twice extracted by using dichloromethane (25 ml), all the resulting dichloromethane solution was combined together to be dried over sodium sulfate, and then the solvent was distilled off. The resulting powder was recrystallized by using pentane to obtain (1-ethyl-2,3,4,5-tetramethylcyclopentadienyl)(pentamethylcyclopentadienyl hafniumdichloride) (157 mg and 45%).
$^1H$ NMR ($CDCl_3$) : δ 0.94 (t, 3H, J = 7.6 Hz, $CH_2CH_3$), 2. 02 (s, 15H, $C_5(CH_3)_5$), 2.03 (s, 6H, $C_5(CH_3)_4Et$), 2.04 (s, 6H, $C_5(CH_3)_4Et$), 2.46 (q, 2H, J = 7.6 Hz, $CH_2CH_3$).
$^{13}C[^1H]$ NMR ($CDCl_3$) : δ 11. 7, 11. 9, 12.0, 14.4, 19. 9, 120.9, 121.9, 122.1, 127.2.

[Synthetic Example 2]

1-n-butyl-2,3,4,5-tetramethylcyclopentadienyllithium (514 mg), pentamethylcyclopentadienyl hafniumtrichloride (1170 mg) and xylene (15 ml) were added to a reaction vessel and reacted therein under a reflux condition for 2 days. The solvent was distilled off under a reduced pressure, and dichloromethane (50 ml) and 1N-HCl aqueous solution (50 ml) were added to perform a liquid separation. The separated aqueous solution was twice extracted by using dichloromethane (25 ml), all the resulting dichloromethane solution was combined together to be dried over sodium sulfate, and then the solvent was distilled off. The resulting powder was recrystallized by using pentane to obtain (1-n-butyl-2,3,4,5-tetramethylcyclopentadienyl) (pentamethylcyclopentadienyl hafniumdichloride) (590 mg and 38%).

$^1H$ NMR ($CDCl_3$): δ0.90 (t, 3H, J = 6.7 Hz, $CH_2CH_3$), 1.22-1. 32 (m, 4H, $CH_2CH_2CH_3$), 2.02 (s, 27H, $C_5(CH_3)_5$ and $C_5(CH_3)_4{}^nBu$), 2.43 (m, 2H, $CH_2CH_2CH_2CH_3$).
$^{13}C[^1H]$ NMR ($CDCl_3$) ; δ 11.9, 11.9, 11.9, 14.1, 23.1, 26.7, 32.4, 121.0, 121.8, 121.9, 126.3.

[Synthetic Example 3]

1-isobutyl-2,3,4,5-tetramethylcyclopentadienyllithium (741 mg), pentamethylcyclopentadienyl hafniumtrichloride (1511 mg) and xylene (35 ml) were added to a reaction vessel and reacted therein under a reflux condition for 2 days. The solvent was distilled off under a reduced pressure, and dichloromethane (50 ml) and 1N-HCl aqueous solution (50 ml) were added to perform a liquid separation. The separated aqueous solution was twice extracted by using dichloromethane (25 ml), all the resulting dichloromethane solution was combined together to be dried over sodium sulfate, and then the solvent was distilled off. The resulting powder was recrystallized by using pentane to produce (1-isobutyl-2,3,4,5-tetramethylcyclopentadienyl) (pentamethylcyclopentadienyl hafniumdichloride) (1090 mg and 54%).
$^1H$ NMR ($CDCl_3$): 5 0.82 (d, 6H, J = 6.6 Hz, $CH(CH_3)_2$), 1.60-1.79 (m, 1H, $CH(CH_3)_2$), 2.02 (s, 6H, $C_5(CH_3)_4{}^iBu$), 2.03 (s, 15H, $C_5(CH_3)_5$), 2.04 (s, 6H, $C_5(CH_3)_4iBu$) ), 2.35 (d, 2H, J = 7.5 Hz, $CH_2CH(CH_3)_2$).
$^{13}C[^1H]$ NMR ($CDCl_3$): δ 12.0, 12.6, 22.8, 29.7, 35.8, 121.4, 121.9, 125.6.

[Synthetic Example 4]

1-ethyl-2,3,4,5-tetramethylcyclopentadienyllithium (444 mg), hafnium tetrachloride (502 mg), and xylene (15 ml) were added to a reaction vessel and reacted therein under a reflux condition for 2 days. The solvent was distilled off under a reduced pressure, and dichloromethane (50 ml) and 1N-HCl aqueous solution (50 ml) were added to perform a liquid separation. The separated aqueous solution was twice extracted by using dichloromethane (25 ml), all the resulting dichloromethane solution was combined together to be dried over sodium sulfate, and then the solvent was distilled off. The resulting powder was recrystallized by using pentane to produce bis(1-ethyl-2,3,4,5-tetramethylcyclopentadienyl) hafniumdichloride (334 mg and 43%).
$^1H$ NMR ($CDCl_3$): δ 0.92 (t, 6H, J = 7.6 Hz, $CH_2CH_3$), 2.01 (s, 12 H, $C_5(CH_3)_4Et$), 2.02 (s, 12H, $C_5(CH_3)_4Et$), 2.45 (q, 4H, J = 7.6 Hz, $CH_2CH_3$).
$^{13}C[^1H]$ NMR ($CDCl_3$): δ 11. 7, 12.0, 14.4, 20.0, 120.9, 122.2, 127.1.

[Synthetic Example 5]

1-n-butyl-2,3,4,5-tetramethylcyclopentadienyllithium (1870 mg), hafnium tetrachloride (1620 mg) and xylene (25 ml) were added to a reaction vessel and reacted therein under a reflux condition for 2 days. The solvent was distilled off under a reduced pressure, and dichloromethane (50 ml) and 1N-HCl aqueous solution (50 ml) were added to perform a liquid separation. The separated aqueous solution was twice extracted by using dichloromethane (25 ml), all the resulting dichloromethane solution was combined together to be dried over sodium sulfate, and then the solvent was distilled off. The resulting powder was recrystallized by using pentane to obtain bis(1-n-butyl-2,3,4,5-tetramethylcyclopentadienyl) hafniumdichloride (1602 mg and 39%).
$^1H$ NMR ($CDCl_3$): δ 0.89 (t, 6H, J = 6.7 Hz, $CH_2CH_3$), 1.07-1.39 (m, 8H, $CH_2CH_2CH_3$), 2.01 (overlapped s, 24H, $C_5(CH_3)_4{}^nBu$), 2. 41 (t, 4H, J = 7.1 Hz, $CH_2CH_2CH_2CH_3$).
$^{13}C[^1H]$ NMR ($CDCl_3$): δ 12.0, 14.1, 23.1, 26.8, 32.4, 121.0, 122.0, 126.3.

[Synthetic Example 6]

1-isobutyl-2,3,4,5-tetramethylcyclopentadienyllithium (1940 mg), hafnium tetrachloride (1690 mg) and xylene (20 ml) were added to a reaction vessel and reacted therein under a reflux condition for 2 days. The solvent was distilled off under a reduced pressure, and dichloromethane (50 ml) and 1N-HCl aqueous solution (50 ml) were added to perform a liquid separation. The separated aqueous solution was twice extracted by using dichloromethane (25 ml), all the resulting dichloromethane solution was combined together to be dried over sodium sulfate, and then the solvent was distilled off. The resulting powder was recrystallized by using pentane to obtain bis(1-isobutyl-2,3,4,5-tetramethylcyclopentadienyl) hafniumdichloride (1171 mg and 37%).
$^1H$ NMR ($CDCl_3$): δ 0.81 (d, 12H, J = 6.6 Hz, $CH(CH_3)_2$), 1.59-1.78 (m, 2H, $CH(CH_3)_2$), 1.94 (s, 12H, $C_5(CH_3)_4{}^iBu$), 1.96 (s, 12H, $C_5(CH_3)_4{}^iBu$), 2.31 (d, 4H, J = 7.3 Hz, $CH_2CH(CH_3)_2$).
$^{13}C[^1H]$ NMR ($CDCl_3$): δ 12.1, 12.7, 22.8, 29.6, 36.0, 123.1, 123.6, 127.3.

[Synthetic Example 7]

1-isobutyl-2,3,4,5-tetramethylcyclopentadienyllithium (2310 mg), zirconium tetrachloride (1460 mg) and xylene (20 ml) were added to a reaction vessel and reacted therein under a reflux condition for 2 days. The solvent was distilled off under a reduced pressure, and dichloromethane (50 ml) and 1N-HCl aqueous solution (50 ml) were added to perform a liquid separation. The separated aqueous solution was twice extracted by using dichloromethane (25 ml), all the resulting dichloromethane solution was combined together to be dried over sodium sulfate, and then the solvent was distilled off. The resulting powder was recrystallized by using pentane to obtain bis(1-isobutyl-2,3,4,5-tetramethylcyclopentadienyl) zirconium dichloride (1420 mg and 44%).
$^1$H NMR ($CDCl_3$): δ 2.31 (d, 4H, J = 7.3 Hz, $CH_2CH(CH_3)_2$), 1.96 (s, 12H, $C_5(CH_3)_4{}^iBu$), 1.94 (s, 12H, $C_5(CH_3)_4{}^iBu$), 1.59-1.78 (m, 2H, $CH(CH_3)_2$), 0.81 (d, 12H, J = 6.6 Hz, $CH(CH_3)_2$).
$^{13}$C[$^1$H] NMR ($CDCl_3$): δ 127.3, 123.6, 123.1, 36.0, 29.6, 22.8, 12.7, 12.1.

[Comparative Example 1]

20 ml of benzene was put into a 50 ml glass autoclave that was sufficiently purged with nitrogen, and a liquid phase and a gas phase were saturated by using propylene (100 kPa). Then, 0.008 mmol of $(Me_5C_5)_2HfMe$ (tetrahydrothiophene) was added to perform a dimerization reaction. After the reaction was performed under a propylene gas atmosphere (gage pressure of 100 kPa) at 50°C for 30 min, a small amount of methanol was added to terminate the reaction. After completion of the reaction, a component having a low-boiling point (having 10 or less carbon atoms) was separated from a component having a high-boiling point under a reduced pressure, and analysis was performed by using gas chromatography. The catalytic activity was 0.7 g-product /(mmol-Hf·h), and the selectivity of 4-methyl-1-pentene to the product was 33%.

[Comparative Example 2]

16 ml of benzene was put into a 50 ml glass autoclave that was sufficiently purged with nitrogen, and a liquid phase and a gas phase were saturated by using propylene (100 kPa). Next, 1.25 mmol of methyl aluminoxane (MAO, 1M toluene solution) in terms of aluminum atom was added and 0.008 mmol of decamethylhafnocene dichloride (4 mM) was then added to perform the dimerization reaction. After the reaction was performed under a propylene gas atmosphere (gage pressure of 100 kPa) at 50°C for 30 min, a small amount of methanol was added to terminate the reaction. After completion of the reaction, a component having a low-boiling point (having 10 or less carbon atoms) was separated from a component having a high-boiling point at a reduced pressure, and analysis was performed by using gas chromatography. The catalytic activity was 719 g-product /(mmol-Hf·h), and the selectivity of 4-methyl-1-pentene to the product was 35%.

[Example 1]

14.5 ml of benzene was put into a 50 ml glass autoclave that was sufficiently purged with nitrogen, and a liquid phase and a gas phase were saturated by using propylene (100 kPa). Next, 0.5 mmol of triisobutyl aluminum (1M benzene solution) and 0.008 mmol of decamethylhafnocene dichloride (4 mM benzene solution) were added and 0.009 mmol of triphenylcarbenium tetrakis(pentafluorophenyl)borate (3 mM benzene solution) was then added thereto to perform the dimerization reaction. After the reaction was performed under a propylene gas atmosphere (gage pressure of 100 kPa) at 50°C for 30 min, a small amount of methanol was added to terminate the reaction. After completion of the reaction, a component having a low-boiling point (having 10 or less carbon atoms) was separated from a component having a high-boiling point under a reduced pressure, and analysis was performed by using gas chromatography. The catalytic activity was 820 g-product /(mmol-Hf·h), and the selectivity of 4-methyl-1-pentene to the product was 39%.

[Examples 2 to 13]

Examples 2, 4, 6, 8, 10 and 12 were performed under the same condition as Comparative Example 2, except that the compound described in Table 1 was used instead of the transition metal compound. Furthermore, Examples 3, 5, 7, 9, 11 and 13 were performed under the same condition as in Example 1, except that the compound described in Table 1 was used instead of the transition metal compound. The results are shown in Table 1.

[TABLE 1]

| Example | Synthetic Example | Transition metal compound* | Additives | Activity (g-product/(mmol-Hf·h)) | Selectivity of 4-methyl 1-pentene (%) |
|---|---|---|---|---|---|
| Example 2 | Synthetic Example 1 | a | MAO | 942 | 34 |
| Example 3 | | | $Ph_3CB(C_6F_5)_4/iBu_3Al$ | 891 | 43 |
| Example 4 | Synthetic Example 2 | b | MAO | 883 | 40 |
| Example 5 | | | $Ph_3CB(C_6F_5)_4/iBu_3Al$ | 862 | 50 |
| Example 6 | Synthetic Example 3 | c | MAO | 860 | 41 |
| Example 7 | | | $Ph_3CB(C_6F_5)_4/iBu_3Al$ | 764 | 51 |
| Example 8 | Synthetic Example 4 | d | MAO | 831 | 43 |
| Example 9 | | | $Ph_3CB(C_6F_5)_4/iBu_3Al$ | 794 | 48 |
| Example 10 | Synthetic Example 5 | e | MAO | 450 | 54 |
| Example 11 | | | $Ph_3CB(C_6F_5)_4/iBu_3Al$ | 432 | 60 |
| Example 12 | Synthetic Example 6 | f | MAO | 311 | 59 |
| Example 13 | | | $Ph_3CB(C_6F_5)_4/iBu_3Al$ | 305 | 62 |

*

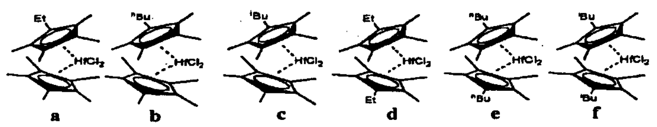

a b c d e f

[Example 14]

The reaction was performed through the same procedure as in Example 1, except that the transition metal compound, which is obtained in Synthetic Example 7 and represented by the following general formula g, was used instead of decamethylhafnocene dichloride. The catalytic activity was 233 g-product/(mmol-Zr·h), and the selectivity of 4-methyl-1-pentene to the product was 45%.

[Chem.10]

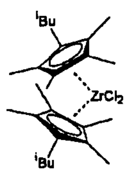

g

Industrial Applicability

The catalyst for dimerizing propylene of the invention has excellent activity and also has high selectivity for production of 4-methyl-1-pentene. Therefore, the catalyst for dimerizing propylene and the method of dimerizing propylene of the invention are expected to make a substantial contribution to industrial fields wherein the efficient production of 4-methyl-1-pentene is required.

## Claims

1. A catalyst for dimerizing propylene, comprising:

   a transition metal complex [A1] that is represented by the following general formula (I):

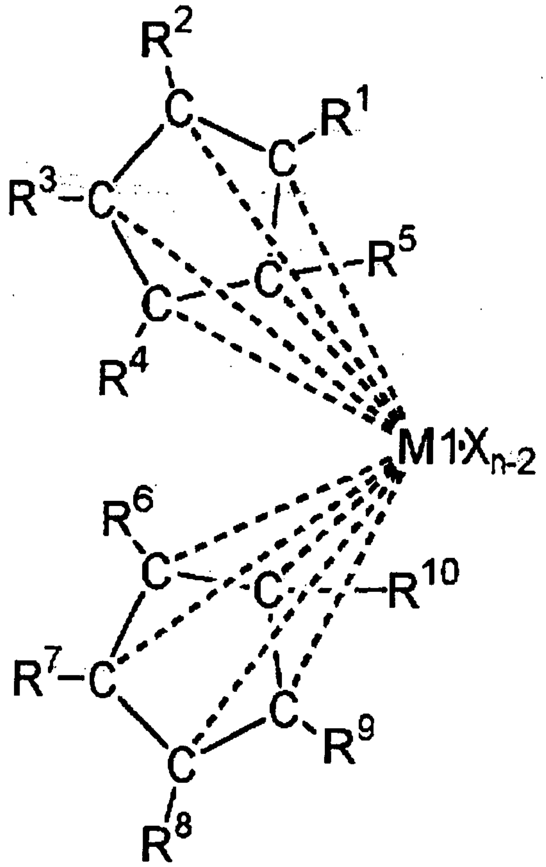

(I)

wherein $R^1$ to $R^{10}$ may or may not be the same and are each a halogen atom, a hydrocarbon group, a heterocyclic compound residue, an oxygen-containing group, a nitrogen-containing group, a boron-containing group, an aluminum-containing group, a sulfur-containing group, a phosphorus-containing group, a silicon-containing group, a germanium-containing group or a tin-containing group, while two or more of the $R^1$ to $R^{10}$ may be linked to each other, and at least one of the $R^1$ to $R^{10}$ is a group other than a methyl group; M1 represents a transition metal atom of Groups III to XI of the periodic table other than lanthanoid atoms and actinoid atoms; n represents a valence of M1; X represents a hydrogen atom, a halogen atom, a hydrocarbon group, an oxygen-containing group, a sulfur-containing group, a nitrogen-containing group, a boron-containing group, an aluminum-containing group, a phosphorus-containing group, a halogen-containing group, a heterocyclic compound residue, a silicon-containing group, a germanium-containing group, or a tin-containing group, while groups represented by X may or may not be the same with each other and they may be linked to each other to form a ring; and each of dotted lines represents a coordinate bond.

2. The catalyst for dimerizing propylene according to claim 1, wherein the hydrocarbon groups of the $R^1$ to $R^{10}$ of the

transition metal complex [A1] that is represented by the general formula (I) are each a hydrocarbon group having 1 to 30 carbon atom(s).

**3.** The catalyst for dimerizing propylene according to claim 1 or 2, wherein M1 of the transition metal complex [A1] that is represented by the general formula (I) is a transition metal atom of Group IV or V of the periodic table.

**4.** The catalyst for dimerizing propylene according to claim 1, wherein the $R^1$ to $R^{10}$ of the transition metal complex [A1] that is represented by the general formula (I) are each a hydrocarbon group having 1 to 20 carbon atom(s) and M1 is a hafnium atom.

**5.** The catalyst for dimerizing propylene according to any one of claims 1 to 4, comprising the transition metal complex [A1] represented by the general formula (I) and at least one compound [B1] selected from the group consisting of an organometallic compound (b1-1), an organoaluminumoxy compound (b1-2) and a compound (b1-3) which reacts with the transition metal complex [A] to form an ion pair.

**6.** A catalyst for dimerizing propylene, comprising:

a transition metal complex [A2] that is represented by a general formula (II):

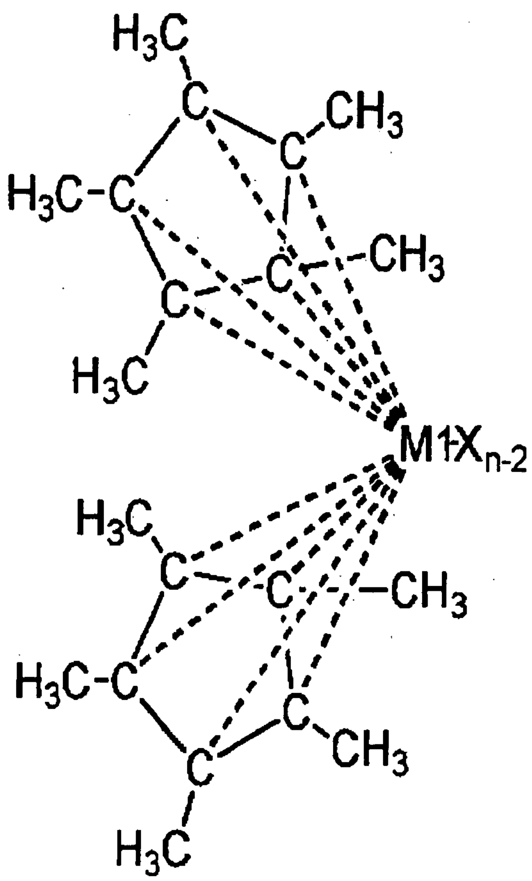

(II)

wherein, M1 represents a transition metal atom of Groups III to XI of the periodic table other than lanthanoid atoms and actinoid atoms; n represents a valence of M1; X represents a hydrogen atom, a halogen atom, a hydrocarbon group, an oxygen-containing group, a sulfur-containing group, a nitrogen-containing group, a boron-containing group, an aluminum-containing group, a phosphorus-containing group, a halogen-containing group, a heterocyclic compound residue, a silicon-containing group, a germanium-containing group or a tin-containing group, while groups represented by X may or may not be the same with each other and they may be linked to each other to form a ring; and each of dotted lines represents a coordinate bond,

and at least one compound [B2] that is selected from the group consisting of an organometallic compound (b2-1) and a compound (b2-3) which reacts with the transition metal complex [A2] to form an ion pair.

**7.** The catalyst for dimerizing propylene according to claim 6, wherein M1 of the transition metal complex [A2] that is represented by the general formula (II) is a hafnium or zirconium atom.

**8.** The catalyst for dimerizing propylene according to any one of claims 1 to 7, further comprising a carrier [C].

**9.** A method of producing 4-methyl-1-pentene, comprising dimerizing propylene in the presence of the catalyst for dimerizing propylene of any one of claims 1 to 8.

**INTERNATIONAL SEARCH REPORT**

International application No.
PCT/JP2006/302077

A. CLASSIFICATION OF SUBJECT MATTER
**B01J31/22**(2006.01), **C07C2/22**(2006.01), **C07C11/113**(2006.01), C07B61/00 (2006.01)

According to International Patent Classification (IPC) or to both national classification and IPC

B. FIELDS SEARCHED

Minimum documentation searched (classification system followed by classification symbols)
B01J31/22, C07B61/00, C07C2/22, C07C11/113

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2006 |
| Kokai Jitsuyo Shinan Koho | 1971-2006 | Toroku Jitsuyo Shinan Koho | 1994-2006 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X<br>Y | JP 1-207248 A (Idemitsu Kosan Co., Ltd.), 21 August, 1989 (21.08.89), Claims 1 to 3; page 3, lower left column, line 1 to upper right column, line 5; page 5, upper right column, line 11 to lower right column, line 1; lower right column, lines 7 to 9; page 7, lower right column, lines 14 to 16; examples 18, 20 to 22; Table 1 & JP 8-109214 A & US 4814540 A & EP 268214 A1 | 1-9<br>5,6 |
| Y | JP 5-39229 A (Idemitsu Kosan Co., Ltd.), 19 February, 1993 (19.02.93), Claims 1 to 3; Par. Nos. [0009], [0013], [0020] to [0025] (Family: none) | 5,6 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 06 April, 2006 (06.04.06) | 18 April, 2006 (18.04.06) |
| Name and mailing address of the ISA/<br>Japanese Patent Office<br><br>Facsimile No. | Authorized officer<br><br>Telephone No. |

Form PCT/ISA/210 (second sheet) (April 2005)

**INTERNATIONAL SEARCH REPORT**

International application No.
PCT/JP2006/302077

C (Continuation). DOCUMENTS CONSIDERED TO BE RELEVANT

| Category* | Citation of document, with indication, where appropriate, of the relevant passages | Relevant to claim No. |
|---|---|---|
| A | JP 63-51340 A (Shell Internationale Research Maatschappij B.V.), 04 March, 1988 (04.03.88), Full text & US 4658078 A & EP 257696 A1 | 1-9 |
| A | JP 6-316538 A (Shell Internationale Research Maatschappij B.V.), 15 November, 1994 (15.11.94), Full text & EP 613873 A3 | 1-9 |

Form PCT/ISA/210 (continuation of second sheet) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*


### Patent documents cited in the description

- US 4695669 A
- JP 2078687 A
- JP 1501950 A
- JP 1502036 A
- JP 3179005 A
- JP 3179006 A
- JP 3207703 A
- JP 3307704 A
- US 5321106 A

### Non-patent literature cited in the description

- *Chemical Reviews,* 1991, vol. 91, 613
- *Chemistry Letter,* 1991, 1525-1528
- *Organometallics,* 1992, vol. 11, 362-369
- *Journal of Molecular Catalysis,* 1990, vol. 62, 277-287
- *Organometallics,* 1985, vol. 4, 97-104
- *Macromolecules,* 2000, vol. 33, 754-759